# NEW EUROPEAN PATENT SPECIFICATION

(11) **EP 1 449 513 B2**
(45) Date of publication and mention of the opposition decision: **13.01.2021**
(45) Mention of the grant of the patent: 10.04.2013
(21) Application number: 03075488.1
(22) Date of filing: 19.02.2003
(51) Int. Cl.: A61K 8/66, A61Q 11/00, A61K 8/27, A61K 8/02

(54) **Dental care product**
Zahnpflegemittel
Produit de soins dentaires

(43) Date of publication of application: 25.08.2004
(73) Proprietor: Unilever PLC, London EC4Y 0DY (GB); Unilever N.V., 3013 AL Rotterdam (NL)
(72) Inventor: Timmer, Christiena Jannie, 6815 HC Arnhem (NL)
(74) Representative: Tansley, Sally Elizabeth

(56) References cited:
- EP-A- 0 277 383
- EP-A- 0 804 920
- EP-A- 0 913 144
- FR-A- 2 822 700
- US-A- 4 871 532
- US-A- 5 607 681
- ANONYMOS: INTERNET ARTICLE, [Online] XP002248544 Retrieved from the Internet: <URL:http://www.walgreens.com/store/produc t.jhtml?PRODID=387485&CATID=100209> [retrieved on 2003-07-09]
- DATABASE CA [Online] CHEMICAL ABSTRACTS SERVICE, COLUMBUS, OHIO, US; K. VARPULEENA ET AL.: "Effects of oral hygiene products containing lactoperoxidase, lysozyme, and lactoferrin on the composition of whole saliva and on subjective oral symptoms in patients with xerostomia" XP002248555 & ACTA ODONTOLOGICA SCANDINAVICA, vol. 54, no. 6, 1996, pages 391-397, Turku, Finland
- ANONYMOS: INTERNET ARTICLE, [Online] XP002248554 Retrieved from the Internet: <URL:http://search.atomz.com/search/?sp-i= 1&sp-q=lactoperoxidase&sp-a=sp1002a36c&sp- p=all&sp-s=1&sp-f=ISO-8859-1> [retrieved on 2003-07-18]
- M. MORRISON ET AL.: "Lactoperoxidase V. Identification and Isolation of Lactoperoxidase from Salivary Gland", ARCHIVES OF BIOCHEMISTRY AND BIOPHYSICS, vol. 111 , - 1965, pages 126-133,
- J. TENOVUO ET AL.: "Concentration of Thiocyanate and Ionizable Iodine in Saliva of Smokers and Nonsmokers", J DENT RES, - 1976, pages 661-663,
- C. HAMON ET AL.: "A Peroxidase-mediated Streptococcus Mitis-dependant Antimicrobial System in Saliva", THE JOUNAL OF EXPERIMENTAL MEDICINE, vol. 137, 1973, pages 438-450,
- E. THOMAS ET AL.: "Hypothiocyanite Ion: Detection of the Antimicrobial Agent in Human Saliva", J DENT RES, vol. 59, no. 9, 1980, pages 1466-1472,
- F. KRAUS ET AL.: J BACTERIOL, vol. 73, no. 6, 1957, pages 727-735,

## Description

Disclosed herein is the use of a dental care product having improved properties in treating and preventing diseases in the oral cavity. More in particular, the invention relates to the use of a dental care product which has an improved capacity of preventing acid formation, e.g. in saliva, thereby preventing caries, tooth erosion and other affectations of the mouth caused by acidity.

One of the most widely encountered problem in dental care is the occurrence of caries. Dental caries, sometimes referred to as tooth decay, is attributable to several factors. On the surface of the teeth, a deposit layer is accumulated known as plaque. This deposit consists of micro-organisms, proteinaceous and carbohydrate substances, epithelial cells, and food debris. Plaque often forebodes a phenomenon called calculus, which is a hard deposit that must be removed mechanically. Plaque may also contribute to various pathological conditions of teeth and soft tissue in the mouth. The micro-organisms that occur in the plaque cause decay of food products, leading to the formation of acids thereby reducing the pH in the mouth.

Many solutions have been proposed in literature to inhibit formation of plaque or to remove it after it is deposited on the teeth's surface. Among these are the use of compounds in dentifrices or other dental care products, such as various organic polyphosphonates, p-amino-benzoic acid, benzo-hydroxamic acid, glutaraldehyde, glyoxylic acid and many others. Unfortunately, none of these has been found to lead to quite satisfactory results.

At the surface of the tooth, the pH of the saliva is normally about 7.0 to 7.5. After intake of certain food products, particularly those containing high amounts of sugar, generation of acid occurs. As a result, the pH may be lowered to 5.5-4.5, or even lower. Such low pH's are generally believed to cause tooth decay as under these conditions the calcium compounds which provide the strength to the teeth will dissolve in the acidic saliva.

The time necessary to restore the natural pH is an important factor in dental care for preventing caries. Obviously, the lower the pH of the saliva becomes, the longer it will take to return to a neutral pH. This time period needed to restore the natural pH value of saliva is furthermore strongly dependent on the type of food intake and the frequency of food consumption. It has for instance been found that during consumption of sugars in the form of a tough mass, such as a toffee, a much lower pH is reached than when the same amount of sugar is consumed in the form of a more fibrous food product, such as an apple. In addition to the lower value the pH reaches, the restoration time to normal pH is also much longer in the first situation. Yet another factor in pH restoration is the thickness of the plaque. When the plaque is very thick, the acids formed within it due to decomposition caused by micro-organisms will need longer to diffuse to the surface, resulting in a lower pH and a longer restoration period directly at the surface of the teeth.

The present invention is as defined in the appended claims. It has been found that use of a dental care product comprising said combination of lactoperoxidase and zinc ions effectively reduces bad breath (halitose).

Unless otherwise indicated, all weight percentages mentioned herein are based on the total weight of the dental care product.

As mentioned, one component of a dental care product used according to the invention is the enzyme lactoperoxidase. Lactoperoxidase is a glycoprotein which, in one commercial embodiment, is a lyophilised powder derived from milk, typically bovine milk. Lactoperoxidase is generally present in a dental care product according to the invention in an amount of 0.2 to 4.0 U per gram, and preferably of 2 U per gram of the dental care product.

The zinc ions will generally be incorporated into a dental care product used according to the invention in the form of a salt. The anion with which the zinc ions form the salt may be any orally acceptable anion. Typical examples include fluoride, fluorosilicate, mono-fluorophosphate, chloride, citrate, gluconate, thiocyanate, sulphate, acetate and the like. Preferably, the anion is gluconate or citrate. The amount of zinc ions as Zn in a dental care product used according to the invention is in the range of from 0.0025 to 0.05 wt.%, more preferably from 0.01 to 0.05 wt.%, even more preferably from 0.025 to 0.05 wt.%. Of course, the weight percentage of the zinc salt incorporated into the dental care product will be larger, depending on the molecular weight of the anion in the salt.

A dental care product used according to the invention may further comprise any conventional components usually present in dental care products, such as abrasives or polishing materials, thickening agents, foaming agents, whitening agents, antibacterial proteins, neutralizing agents (bicarbonate), flavouring agents, sweeteners, preservatives and/or other basic ingredients. The choice for these components will depend on the actual nature of the dental care product.

The dental care product may be substantially solid or pasty in character (tooth powders, dental tablets, toothpastes, ordentalgels). Such solid or pasty preparations generally comprise an abrasive or polishing material.

Suitable examples of polishing materials include water-insoluble sodium metaphosphate, potassium metaphosphate, tricalcium phosphate, dehydrated calcium phosphate, anhydrous dicalcium phosphate, calcium pyrophosphate, magnesium orthophosphate, trimagnesium phosphate, aluminium oxide, aluminium oxide hydrate, calcium carbonate, aluminium silicate, zirconium silicate, silica, bentonite and mixtures thereof. Other suitable polishing materials include particulate thermosetting resins as disclosed in US patent 3,070,510, such as melamine-phenolic-, and urea-formaldehydes, and crosslinked polyepoxides and polyesters. Preferred polishing materials include silica having particle sizes of up to 7 microns, a mean particle size of up to 1.1 microns, and a surface area of up to 50,000 cm²/gm, silica gel or colloidal silica, and complex amorphous alkali metal aluminosilicate.

When visually clear gels are desired, a polishing agent of colloidal silica, such as those sold under the trademark SYLOID as Syloid 72 and Syloid 74, or under the trademark SANTOCEL as Santocel 100 and alkali metal aluminosilicate complexes are particularly useful, since they have refractive indices close to the refractive indices of gelling agent-liquid (including water and/or humectant) systems commonly used in dental care products.

The polishing material is typically present in an amount ranging from 10 to 99 wt.%. Preferably, a polishing material is present in an amount from 10 to 75 wt.% in toothpaste, and from 70 to 99 wt.% in tooth powder.

In a toothpaste, the liquid vehicle may comprise water and humectant typically in an amount ranging from 10 to 90 wt.%. Glycerine, propylene glycol, sorbitol, polypropylene glycol, polyethylene glycol and mixtures thereof are typical examples of suitable humectants or carriers. Also advantageous are liquid mixtures of water, glycerine and sorbitol. In clear gels where the refractive index is an important consideration, 3-30 wt.% of water, 0 to 80 wt.% of glycerine, and 20-80 wt.% of sorbitol is preferably employed.

Toothpastes and gels typically comprise a natural or synthetic thickener or gelling agent in proportions of 0.1 to 10 wt.%, preferably 0.5 to 5 wt.%. An example of a suitable thickener is synthetic hectorite, a synthetic colloidal magnesium alkali metal silicate complex clay available for examples under the trademark LAPONITE (e.g. CP, SP 2002, D) marketed by Laporte Industries Limited. Other suitable examples of thickeners include Irish moss, gum tragacanth, starch, polyvinyl pyrrolidone, hydroxyethyl propyl cellulose, hydroxybutyl methyl cellulose, hydroxypropyl methyl cellulose, hydroxyethyl cellulose (e.g. available as Natrosol), sodium carboxymethyl cellulose, and colloidal silica such as finely ground Syloid TM.

The vehicle or carrier in a tablet or lozenge is preferably a non-cariogenic solid water soluble polyhydric alcohol (polyol), such as mannitol, xylitol, sorbitol, maltitol, a hydrogenated starch hydrolysate, Lycasin, hydrogenated glucose, hydrogenated disaccharides, and hydrogenated polysaccharides, in an amount of 90-98 wt.%. Solid salts such as sodium carbonate, sodium chloride, potassium bicarbonate, or potassium chloride may totally or partially replace the polyol carrier. Tabletting lubricants, in minor amounts of 0.1 to 5 wt.%, may be incorporated into the tablet or lozenge formulation to facilitate the preparation of both the tablets and lozenges. Suitable lubricants include vegetable oils such as coconut oil, magnesium stearate, aluminium stearate, talc, starch and carbowax.

Formulation of a dental care product in the form of a lozenge or chewing gum may involve stirring any of the components set forth above into a warm gum base or coating the outer surface of a gum base. Illustrative of suitable gum bases are jelutone, rubber latex, vinylite resins, and the like, preferably in combination with conventional plasticizers or softeners, sugar or other sweeteners or carbohydrates such as glucose, sorbitol and the like.

Lozenge formulations may optionally comprise 2 wt.% gum as barrier agent to provide a shiny surface as opposed to a tablet which has a smooth finish. Suitable non-cariogenic gums include Kappa carrageenan, carboxymethyl cellulose, hydroxyethyl cellulose, Gantrez™, and the like. The lozenge or tablet may optionally be coated with a coating material such as a wax, shellac, carboxymethyl cellulose, polyethylene maleic anhydride copolymer or Kappa carrageneean to further increase the dissolution time of the tablet or lozenge in the mouth. The uncoated tablet or lozenge is slow dissolving, providing a sustained release rate of the active ingredients. Accordingly, the solid dose tablet or lozenge compositions according to the invention afford a relatively longer contact time between active ingredients and oral tissues and structures.

Any suitable flavouring or sweetening material may also be employed. Examples of suitable flavouring constituents are flavouring oils, e.g. oil of spearmint, peppermint, wintergreen, sassafras, clove, sage, eucalyp-tus, marjoram, cinnamon, lemon, and orange, and methyl salicylate. Suitable sweetening agents include sucrose, lactose, maltose, sorbitol, xylitol, sodiuk cyclamate, perillartine, APM (aspartyl phenyl alanine methyl ester), saccharine, and the like. Suitably, flavouring and sweetening agents may together comprise from 0.1 to 5 wt.% or more of the dental care product.

In addition to lactoperoxidase and zinc ions, a dental care product may comprise one or more other materials that are considered as active components in oral care products, such as fluoride ion-providing agents, phosphates, such as trimetaphosphates and diammoniumphosphates, enzymes, anti-tartar agents such as pyrophosphates, phosphonates and/or other antiplaque ingredients, such as triclosan, chlorhexidine, bromochlorophene and sanquinarine, antibacterial proteins, such as lanthibiotics (preferably nisin), agents for sensitive teeth, such as specific alkali metal salts like strontium salts and/or potassium nitrate or citrate, wound healing agents, such as allantoin, chlorophyll, tocopherol and herbal extracts, activity enhancing agents, or boosters, as Gantrez™ and some polymers, and specific ingredients as urea, xylitol, silicones, quaternary ammonium compounds and mixtures thereof. These components, as well as the amounts in which they may be used, are known in the art. If they are incorporated, they should be used in amounts which do not substantially adversely affect the properties and characteristics desired.

The presence of one or more fluoride ion-providing agents in a dental care product according to the invention is particularly preferred. The use of fluoride ions, or fluoride ion providing compounds as anti-caries agents is well known in the art. Suitable compounds may be slightly soluble in water or may be fully water soluble. They are characterized by their ability to release fluoride ions in water and by freedom from undesired reaction with other compounds of a dental care prod uct according to the invention. Particularly, the use of alkali metal fluorides, more in particular sodium fluoride, results in improved activity of a dental care product.

Among suitable compounds yielding fluoride ions are inorganic fluoride slats, such as soluble alkali metal and alkaline earth metal salts, for example sodium fluoride, potassium fluoride, barium fluoride or calcium fluoride, and ammonium fluoride, or a copper fluoride, zinc fluoride, sodium fluorosilicate, ammonium fluorosilicate, sodium fluorozirconate, sodium monofluorphosphate, aluminium mono- or di-fluorophosphate and fluorinated sodium calcium pyrophosphate. Alkali metal and tin fluoride, such as sodium and stannous fluorides, sodium mono-fluorophosphate, organic fluorides, such as aminfluoride and mixtures thereof, are preferred.

The amount of fluoride ion-providing compounds is dependent to some extent upon the type of compound, its solubility, and the type of dental care product, but it must be a non-toxic, effective amount, generally 0.005 to 3.0 wt.% in the dental care product, an amount of such compound which releases up to 5,000 ppm of fluoride ions by weight of the dental care product is considered satisfactory. Any suitable minimum amount of such compound may be used, but it is preferable to employ sufficient compound to release 300 to 2,000 ppm, more preferably 800 to 1,500 ppm of fluoride ions. Typically, in the case of alkali metal fluorides and stannous fluoride, this component is present in an amount up to 2 wt.%, and preferably in the range of 0.05-1 wt.%. In the case of sodium mono-fluorophosphate, the component may be present in an amount of 0.1-3 wt.%. In dental care products such as lozenges and chewing gums, the fluoride ion-providing compound is typically present in an amount sufficient to release up to 500 ppm, preferably 25 to 300 ppm by weight of fluoride ions. Generally, 0.005 to 1.0 wt.% of such compound is present.

Other enzymes that can be used to advantage in addition to lactoperoxidase in a dental care product used according to the invention include those described in US patents 4,150,113, 4,178,362 and 4,781,532 and European patent application 0 133 736. Particular enzymes that are preferably used are hydrogen peroxide producing enzyme systems, which have advantageous effects on the reduction of oral bacterial activity. Specific examples include oxidoreductases, such as glucose oxidase, galactose oxidase, urate oxidase, cholineoxidase, D-amino acid oxidase, D-glutamate oxidase, glycine oxidase, glycollage oxidase, L-sorbose oxidase, alcohol oxidase, or amine oxidase, carbohydrases, such as α-amylase, glucoamylase or amylglucosidase, cellulase, dextranase, invertase, or α- or β-glucosidase, hydrolases and proteases. Preferably used are glucose oxidase, dextranase, lactoferrin, lysozyme, amyloglucosidase, and/or mutanase.

In order to enhance the activity of the lactoperoxidase, and of oxdioreductases if present, it is preferred that a dental care product used according to the invention comprises a thiocyanate salt (SCN-). Preferred thiocyanate salts are alkali metal salts and alkaline earth metal salts, such as potassium thiocyanate or sodium thiocyanate. Thiocyanate salts are generally included in an amount in the range of from 0.005 to 1 wt.%, dependent on the type of dental care product. For toothpastes, mouth washes and chewing gums the amount will preferably lie in the range of from 0.005 to 0.1 wt.%. For lozenges and effervescent tablets the amount preferably is in the range of from 1 to 10 mg/tablet.

The pH of a dental care product used according to the invention is generally in the range of from 4.5 to 10, and preferably from 5.5 to 8. The pH can be controlled with acid (e.g. citric acid or benzoic acid) or base (e.g. sodium hydroxide), or be buffered using a neutralizing agent (e.g. sodium bicarbonate, citrate, benzoate, carbonate, disodium hydrogen phosphate, or sodium dihydrogen phosphate).

It is furthermore preferred that colostrum is included in a dental care product used according to the invention. Colostrum typically consists of a mixture of lacteal secretions and constituents of blood serum, notably immunoglobulins and other proteins, that accumulate in the mammary gland during the prepartum dry period and can be harvested immediately preceding or following parturition. The first six milkings collected during the period of transition from colostrum to milk are typically referred to as colostrum, in the case of cows as bovine colostrums, which is preferred in accordance with the invention. More in particular, colostrums is generally defined in the art as the mammary secretion occurring in the period starting some days before delivery until some days after delivery. In the context of the invention, the term colostrum is intended to also include milk from animals, notably cows, which are treated with specific micro organisms such as S-mutans and other oral care selected pathogenic bacteria to produce a colostrum-type milk for longer periods (i.e. up to a year) after delivery rich in immunoglobulins.

Colostrum may be added in dried form, such as lyophilised or spray dried, or in the form of an extract, such as a concentrate or purified form. The amount in which colostrums is preferably used depends on the type of dental care product being prepared. Generally, the amount will lie within the range of from 0.001 to 50 wt.%, preferably from 0.01 to 25 wt.%., more preferably from 0.1 to 5 wt.%. For toothpaste, the amount is preferably between 1 and 50 wt.%, more preferably between 5 and 15 wt.%; and for chewing gums the amount is preferably between 0.1 and 10 wt.%, more preferably between 0.5 and 3 wt.%.

A dental care product used according to the invention can suitably be prepared using conventional methods and techniques for preparing such products, wherein the lactoperoxidase and zinc ions, typically in the form of a zinc salt, are included during the preparation together with other active ingredients, or are added to the finished dental care product. Any relevant process parameters such as temperatures and pressures may suitably be chosen within conventional ranges.

The invention will now be elucidated by the following, non-restrictive examples.

### Example I

A toothpaste was prepared according to a method known in the art from the following ingredients. All amounts are percentages by weight unless otherwise indicated. The formulated product had a pH of 5.8-6.2.

| | |
|---|---|
| Sorbitol | 15 |
| Silica | 22 |
| Ethoxylated fatty acid | 3 |
| Carraghene | 1.5 |
| Sodium benzoate | 0.15 |
| Aroma | 0.6 |
| Sodium saccharinate | 0.1 |
| Glycerol | 3 |
| Sodium fluoride | 0.33 |
| Disodium phosphate | 0.5 |
| Citric acid | 0.23 |
| Dried bovine colostrum | 1 |
| Amyloglucosidase | 7 U/g |
| Glucose oxidase | 6 U/g |
| Zinc gluconate | 0.25 |
| Xylitol | 10 |
| Potassium thiocyanate | 0.02 |
| Lactoperoxidase | 2 U/g |
| Lysozyme | 0.01 |
| Lactoferrin | 0.01 |
| Dextranase | 16 U/g |
| Mutanase | 16 U/g |
| Water | to 100% |

### Example II

A toothpaste was prepared according to a method known in the art from the following ingredients. All amounts are percentages by weight unless otherwise indicated. The formulated product had a pH of 5.8-6.2.

| | |
|---|---|
| Sorbitol | 15 |
| Aluminium oxide hydrate | 35 |
| Ethoxylated fatty acid | 3 |
| Carraghene | 1.75 |
| Sodium benzoate | 0.15 |
| Aroma | 0.6 |
| Sodium saccharinate | 0.1 |
| Glycerol | 15 |
| Sodium fluoride | 0.33 |
| Disodium phosphate | 0.5 |
| Citric acid | 0.23 |
| Dried bovine colostrum | 0.1 |
| Amyloglucosidase | 7 U/g |
| Glucose oxidase | 6 U/g |
| Zinc gluconate | 0.25 |
| Xylitol | 10 |
| Potassium thiocyanate | 0.02 |
| Lactoperoxidase | 2 U/g |
| Lysozyme | 0.01 |
| Lactoferrin | 0.01 |
| Dextranase | 16 U/g |
| Mutanase | 16 U/g |
| Water | to 100% |

### Example III

A mouth wash was prepared according to a method known in the art from the following ingredients. All amounts are percentages by weight unless otherwise indicated. The formulated product had a pH of 5.8-6.2.

| | |
|---|---|
| Ethoxylated fatty acid | 1 |
| Sorbitol | 5 |
| Sodium benzoate | 0.55 |
| Aroma | 0.05 |
| Sodium saccharinate | 0.1 |
| Glycerol | 20 |
| Sodium fluoride | 0.05 |
| Disodium phosphate | 0.5 |
| Citric acid | 0.23 |
| Dried bovine colostrum | 0.01 |
| Amyloglucosidase | 3 U/g |
| Glucose oxidase | 6 U/g |
| Zinc gluconate | 0.08 |
| Xylitol | 3.5 |
| Potassium thiocyanate | 0.02 |
| Lactoperoxidase | 2 U/g |
| Lysozyme | 0.01 |
| Lactoferrin | 0.01 |
| Dextranase | 5 U/g |
| Mutanase | 5 U/g |
| Water | to 100% |

### Example IV

A chewing gum was prepared according to a method known in the art from the following ingredients. All amounts are percentages by weight unless otherwise indicated.

| | |
|---|---|
| Sorbitol | to 100% |
| Mannitol | 5 |
| Aroma | 2 |
| Apartame/potassium acesulphane | 0.2 |
| Lycasin | 1.5 |
| Glycerol | 3 |
| Dried bovine colostrums | 1 |
| Amyloglucosidase | 18 U/g |
| Glucose oxidase | 6 U/g |
| Zinc gluconate | 0.25 |
| Xylitol | 25 |
| Potassium thiocyanate | 0.02 |
| Lactoperoxidase | 2 U/g |
| Lysozyme | 0.01 |
| Lactoferrin | 0.01 |
| Dextranase | 16 U/g |
| Mutanase | 16 U/g |

### Example V

A lozenge was prepared according to a method known in the art from the following ingredients. All amounts are in milligrams unless otherwise indicated.

| | |
|---|---|
| Sorbitol | 1500 |
| Encapsulated aroma | 10 |
| Sodium stearate | 10 |
| Phosphate buffer pH 5.5 | 50 |
| Polyvinyl pyrrolidone (PVP) | 20 |
| Dried bovine colostrum | 500 |
| Amyloglucosidase | 7 U/g |
| Glucose oxidase | 6 U/g |
| Zinc gluconate | 70 |
| Xylitol | 250 |
| Potassium thiocyanate | 5 |
| Lactoperoxidase | 2 U/g |
| Lysozyme | 3 |
| Lactoferrin | 3 |
| Dextranase | 16 U/g |
| Mutanase | 16 U/g |

### Example VI

An effervescent tablet was prepared according to a method known in the art from the following ingredients. All amounts are in milligrams unless otherwise indicated.

| | |
|---|---|
| Sodium carbonate | 40 |
| Encapsulated aroma | 2 |
| Sodium stearate | 10 |
| Citric acid | 50 |
| Polyvinyl pyrrolidone (PVP) | 20 |
| Dried bovine colostrum | 20 |
| Sorbitol | 24 |
| Amyloglucosidase | 70 U/g |
| Glucose oxidase | 60 U/g |
| Zinc gluconate | 37 |
| Potassium thiocyanate | 3 |
| Lactoperoxidase | 2 U/g |
| Lysozyme | 1.5 |
| Lactoferrin | 1.5 |
| Dextranase | 16 U/g |
| Mutanase | 16 U/g |

## Claims

1. The non-therapeutic use of zinc ions to enhance the effect of lactoperoxidase for use in a dental product to reduce halitose, wherein the amount of zinc ions is in the range of from 0.0025 to 0.05%.

2. The non-therapeutic use of lactoperoxidase to enhance the effect of zinc ions for use in a dental product to reduce halitose, wherein the amount of zinc ions is in the range of from 0.0025 to 0.05%.

## Patentansprüche

1. Nichttherapeutische Verwendung von Zinkionen, um die Wirkung von Lactoperoxidase zur Verwendung in einem Zahnpflegeprodukt zur Verringerung von Halitose zu verstärken, wobei die Menge an Zinkionen im Bereich von 0,0025 bis 0,05 % liegt.

2. Nichttherapeutische Verwendung von Lactoperoxidase, um die Wirkung von Zinkionen zur Verwendung in einem Zahnpflegeprodukt zur Verringerung von Halitose zu verstärken, wobei die Menge an Zinkionen im Bereich von 0,0025 bis 0,05 % liegt.

## Revendications

1. Utilisation non thérapeutique d'ions zinc pour améliorer l'effet de la lactoperoxydase pour une utilisation dans un produit bucco-dentaire pour réduire l'halitose, dans laquelle la quantité d'ions zinc est dans la plage de 0,0025 à 0,05 %.

2. Utilisation non thérapeutique de lactoperoxydase pour améliorer l'effet des ions zinc pour une utilisation dans un produit bucco-dentaire pour réduire l'halitose, dans laquelle la quantité d'ions zinc est dans la plage de 0,0025 à 0,05 %.
